# EUROPEAN PATENT APPLICATION

(11) **EP 4 194 090 A1**
(43) Date of publication of application: **14.06.2023**
(21) Application number: 20953415.5
(22) Date of filing: 07.10.2020
(51) Int. Cl.: B01J 35/00, B01J 35/08, B01J 37/04, B01J 37/02, B01J 37/08, B01J 21/06, B01J 29/04, C09K 11/64, C09K 11/02, A61L 9/20

(54) **PRODUCTION METHOD FOR PHOSPHORESCENT PHOTOCATALYST BEADS FOR BREAKING DOWN HARMFUL SUBSTANCES AND FOR ANTI-VIRAL PURPOSES, AND PHOTOCATALYST BEADS OBTAINED THEREBY**

(30) Priority: 11.09.2020 KR 20200116596
(71) Applicant: APC TEC. Co., Ltd., Chungcheongbuk-do 27462 (KR)
(72) Inventor: KIM, Seung Jin, Seoul 05042 (KR)
(74) Representative: BCKIP Part mbB
(86) International application number: PCT/KR2020/013642
(87) International publication number: WO 2022/055013

(57) **Abstract**

The present invention relates to a method of preparing photoluminescent photocatalyst beads for removing harmful substances or viruses present in air, soil, and water, and more particularly to a method of preparing photoluminescent photocatalyst beads that are efficient in decomposing and removing a mixture of hard-to-decompose organic contaminants and removing viruses by preparing a photoluminescent photocatalyst in the form of a bead, and photoluminescent photocatalyst beads obtained from the method.

## Description

### [Technical Field]

The present invention relates to a method of preparing photoluminescent photocatalyst beads for removing harmful substances or viruses present in air, soil, and water, and more particularly, to a method of preparing photoluminescent photocatalyst beads that are efficient in decomposing and removing a mixture of hard-to-decompose organic contaminants and removing viruses by preparing a photoluminescent photocatalyst in the form of a bead, and photoluminescent photocatalyst beads obtained from the method.

### [Background Art]

Recently, interest in indoor air purification is increasing due to yellow dust as well as air pollution, and as a result, the demand for air purifiers is increasing. Various studies are being conducted to effectively purify indoor air polluted by automobile exhaust gases, toxic organic compounds, repulsive odors, and viruses such as the recent coronavirus.

In general, a method using a filter has been mainly used, but recently, a method of purifying air through a photolysis reaction using a photocatalyst is increasing day by day.

A photocatalyst has catalytic activity by absorbing light energy, and oxidatively decomposes environmental pollutants such as organic matter with strong oxidizing power by catalytic activity. That is, the photocatalyst absorbs light (ultraviolet rays) having energy greater than or equal to a band gap, so that electrons transition from a valence band to a conduction band, and holes are formed in the valence band. These electrons and holes are diffused to a surface of the powder and come into contact with oxygen and moisture to cause a redox reaction or recombine to generate heat. That is, electrons in the conduction band reduce oxygen to generate superoxide anions, and holes in the valence band oxidize moisture to form hydroxyl radicals (OH·). Due to the strong oxidizing power of a hydroxyl radical generated by the holes, decomposition, sterilization power, hydrophilicity, and the like of gaseous or liquid organic matter adsorbed on a surface of the photocatalyst, that is, hard-to-decompose organic matter, may be exhibited.

Titanium dioxide (TiOz) is a representative photocatalytic material, which is harmless to the human body, has excellent photocatalytic activity and excellent light corrosion resistance, and is inexpensive. When titanium dioxide receives ultraviolet light, it generates radicals with strong oxidizing power, and these radicals can decompose various environmental pollutants present in water or air into harmless carbon dioxide and water.

Titanium dioxide absorbs and reacts with ultraviolet rays of 388 nm or less to generate electrons and holes, and in this case, ultraviolet light used as a light source may be artificial lighting such as a lamp, an incandescent lamp, a mercury lamp, or a light emitting diode, in addition to sunlight. The electrons and holes generated in the reaction recombine in 10⁻¹² to 10⁻⁹ seconds, but when contaminants or the like are adsorbed on the surface before recombination, they are decomposed by the electrons and holes.
However, since about 2% of light can be used to obtain the band gap energy (wavelength of 380 nm or more) of titanium dioxide powder from sunlight, it is difficult to have smooth catalytic activity in a visible light region (400 to 800 nm), which is the main wavelength range of sunlight.

### [Related Art Document]

### (Patent Documents)

(Patent Document 1) Korean Patent Registration No. 1918398 "Photocatalyst for decomposition of bad odors and harmful air pollutants"
(Patent Document 2) Korean Patent Registration No. 1721027 "Visible light-sensitive photocatalyst composition and lighting device using the same"

### [Disclosure]

### [Technical Problem]

The present invention has been made to solve the above problems, and provides a novel method of preparing photocatalyst beads containing a phosphor that can be used in products such as air purifiers.

The present invention provides beads composed of a phosphor-zeolite-inorganic binder-titanium dioxide obtained by the preparation method, and provides a photoluminescent photocatalyst bead that is photoactive under visible light as well as ultraviolet light as a composite photocatalyst bead including a photocatalytic material titanium dioxide and a phosphor.

Since the photoluminescent photocatalyst bead according to the present invention causes a photolysis reaction by light generated from a fluorescent material even in a dark state by storing light, it can perform an air purifying function even in an environment without light, and has an excellent air purifying effect because it has the form of a bead.

### [Technical Solution]

One embodiment according to the present invention provides a method of preparing photoluminescent photocatalyst beads for decomposition of harmful substances and removal of viruses, including: ball-milling a mixture of a phosphor, zeolite, an inorganic binder, and titanium dioxide for 6 to 10 hours; mixing 10 to 50 parts by weight of water with 100 parts by weight of the ball-milled mixture and kneading; preparing the kneaded product in the form of the beads through a bead filling machine; drying the beads at 100 to 110 °C for 1 to 3 hours; immersing the dried beads in a SiOz sol to coat the beads; subjecting the coated beads to a first heat treatment at 400 to 500 °C for 1 to 2 hours; primarily immersing the first heat-treated beads in a 10% sodium silicate solution; subjecting the beads, which have been primarily immersed, to a second heat treatment at 100 to 150 °C for 1 hour to 2 hours; secondarily immersing the second heat-treated beads in a 10% sodium silicate solution; subjecting the beads, which have been secondarily immersed, to a third heat treatment at 100 to 150 °C for 1 hour to 2 hours; immersing the third heat-treated beads in a TiOz sol to coat the beads with TiOz; and subjecting the TiOz-coated beads to a fourth heat treatment at 400 to 500 °C for 1 to 2 hours.

The inorganic binder according to the present invention may be selected from sodium alginate and sodium silicate. The inorganic binder has excellent visible light transmittance and thus has an advantage of increasing the activity of photocatalyst beads containing a phosphor.

The phosphor according to the present invention is selected from the group consisting of CaAl₂O₄: Eu-based, SrAl₂O₄:Eu-based, BaAl₂O₄: Eu-based, ZnS: Cu-based, CaAl₂O₁₉: Eu-based, SrAl₂O₁₉: Eu-based, BaAl₂O₁₉: Eu-based, BaMgAl₁₀O₁₇: Eu-based, SrMgAAl₁₀O₁₇: Eu-based, and BaMg₂Al₁₀O₁₇: Eu-based phosphors, and is not particularly limited in the present invention.

The zeolite according to the present invention is a powder raw material having the formula (WₘZₙO₂)ₙ·₂O (W is Na, Ca, Ba, Sr, and Z=Si+Al).

The mixing ratio of the phosphor, the zeolite, the inorganic binder, and the titanium dioxide according to the present invention may be 30 to 50% by weight of a phosphor, 30 to 50% by weight of zeolite, 10 to 20% by weight of an inorganic binder, and 1 to 5% by weight of TiOz, and when the mixing ratio is within the above range, photocatalytic efficiency can be increased while having a photocatalytic activation effect using a phosphor.

After drying the photoluminescent photocatalyst beads in the form of beads prepared through a bead filling machine, coating is performed by immersing the beads in a SiOz sol, wherein the SiOz sol according to the present invention is prepared by mixing tetraethyl orthosilicate(TEOS):ethanol:distilled water:nitric acid in a ratio of 2:13.5:11.5:0.3 parts by weight, mixing 0.8 parts by weight of polydimethylsiloxane (PDMS) based on 100 parts by weight of the mixture, stirring at 30 °C for 1 hour, and then aging for 24 hours.

The photoluminescent photocatalyst beads immersed and coated in the SiOz sol are immersed in a 10% sodium silicate solution twice and then dried at 100 to 150 °C for 1 to 2 hours. In this way, in the present invention, performing the process of immersing in the 10% sodium silicate solution and drying twice is made to harden the surface of the photoluminescent photocatalyst beads such that there is no problem in exhibiting the activity of the photocatalyst by preventing the phenomenon in which the beads break or the phosphor is peeled off even if the beads collide with each other.

Next, the photoluminescent photocatalyst beads whose surfaces are firmly treated are subjected to TiOz coating, which is characterized by being immersed and coated in the TiOz sol being stirred at 30 °C for 1 hour, and the TiOz coating according to the present invention is prepared by mixing titanium isopropoxide (TTIP):ethanol:nitric acid:distilled water in a ratio of 2:13.5:0.3:11.5 parts by weight.

The photoluminescent photocatalyst beads according to the present invention, which have been coated with TiOz as described above, are calcined at 400 to 450 °C for 2 to 3 hours to obtain photoluminescent photocatalyst beads for decomposition of harmful substances and removal of viruses according to the present invention.

Another embodiment of the present invention provides photoluminescent photocatalyst beads for decomposition of harmful substances and removal of viruses, prepared by the preparation method.

Another embodiment of the present invention provides a module using the photoluminescent photocatalyst beads for decomposition of harmful substances and removal of viruses obtained by the preparation method according to the present invention, which can be attached to and used in an air purifier, or the like.

### [Advantageous Effects]

According to one embodiment of the present invention, by improving an existing solgel method, beads are made by mixing a phosphor and a photocatalyst, and then post-coating is performed to give the beads firmness, so that there is an advantage in that the photocatalyst and phosphor are not easily peeled off and the effect is sustained.

The photoluminescent photocatalyst beads according to one embodiment of the present invention can act as a light source for activating a photocatalyst by re-emitting not only ultraviolet or visible light irradiated from the outside, but also light already absorbed by the phosphor, so that there is an advantage of improving photolysis reaction efficiency by titanium dioxide.

Since the photoluminescent photocatalyst beads according to the present invention have a spherical shape, a photoreactive specific surface area can be increased, and thus photolysis efficiency can be improved.

By manufacturing a module capable of stacking beads according to one embodiment of the present invention and arranging photoluminescent photocatalyst beads prepared according to the present invention therein, the efficiency of air cleaning in a vehicle can be increased by combining the module with an air filter inside an automobile, and when the module is combined with the outside of an air purifier for general household or industrial use, it has an effect of purifying indoor air and sterilizing viruses.

### [Description of Drawings]

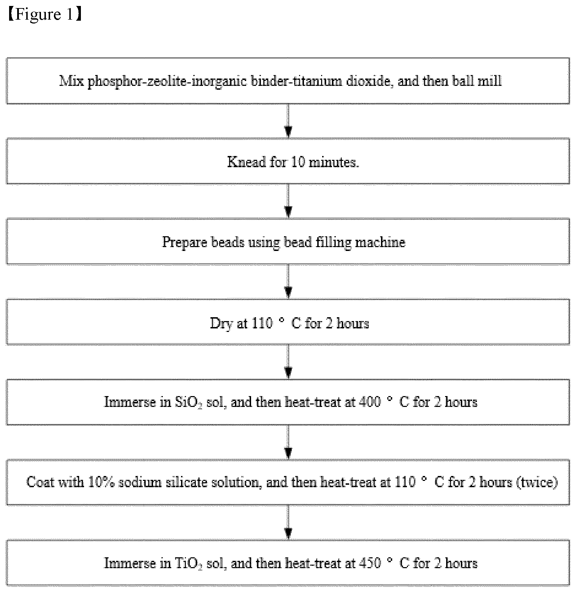

FIG. 1 is a flowchart schematically illustrating a method of preparing photoluminescent photocatalyst beads according to one embodiment of the present invention.

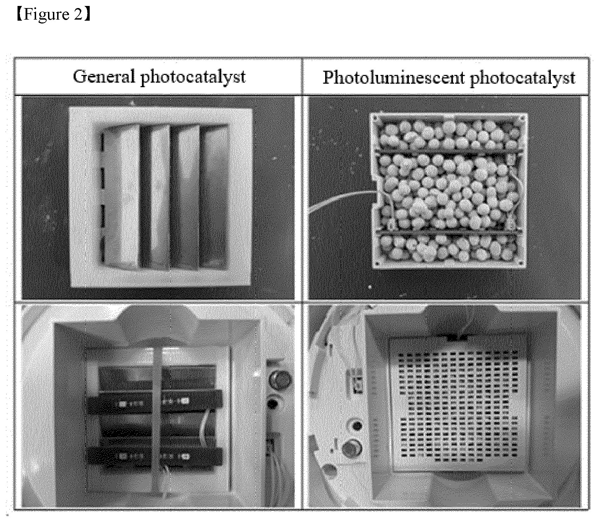

FIG. 2 is a photograph comparing the shapes of a general photocatalyst and photoluminescent photocatalyst beads prepared by the preparation method according to the present invention.
FIG. 3 is a graph showing the results of photolysis experiments using a general photocatalyst and photoluminescent photocatalyst beads according to the present invention.
FIG. 4 is a test report on the harmful gas removal efficiency of an air purifier manufactured using photoluminescent photocatalyst beads according to the present invention.

### [Modes of the Invention]

Hereinafter, preferred embodiments of the present disclosure will be described in more detail. However, the following specific structure or functional descriptions are only illustrated for the purpose of explaining embodiments according to the concept of the present invention, embodiments according to the concept of the present invention can be implemented in various forms, and it should not be understood as being limited to the examples described herein.

### Example

Phosphor: zeolite: sodium alginate:sodium silicate:titanium dioxide were mixed in a wt% ratio of 37:37:8:17:1, and then were ball-milled for 6 hours. 20 kg of water was added to 100 kg of the ball-milled mixture and kneaded for 10 minutes, and the resulting mixture was prepared in the form of beads with a size of 5 mm in a bead filling machine.

The beads prepared as above were dried at 110 °C for 2 hours.

After coating the dried beads by immersing the dried beads in a SiOz sol, a first heat treatment was performed at 400 °C for 2 hours.

The coated beads were primarily immersed in a 10% sodium silicate solution, subjected to a second heat treatment at 110 °C for 1 hour, secondarily immersed in a 10% sodium silicate solution, and then subjected to a third heat treatment at 110 °C for 1 hour.

The heat-treated beads were immersed in a TiOz sol, coated with TiOz, and then heat-treated at 450 °C for 2 hours to obtain photoluminescent photocatalyst beads according to the present invention.

### Comparative Example

A photocatalyst plate was prepared by coating titanium dioxide on a metal plate (SUS) and used in the experiment.

### Experimental Example

### Photolysis experiment

1 drop of acetic acid was added to a 0.3 m³ chamber, diffused for 30 minutes, and followed by photolysis for 2 hours.

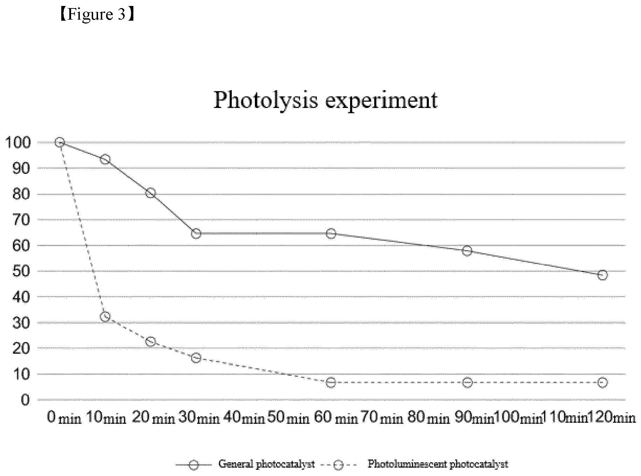

As shown in FIG. 3, it was found that a general photocatalyst according to the Comparative Example exhibited a photolysis effect of about 40% for an initial 30 minutes and a final photolysis effect of 50%.

In contrast, the photoluminescent photocatalyst prepared according to the present invention exhibited a photolysis effect of 85% for 30 minutes and a final photolysis effect of 95%.

As can be seen from the experimental results, it was confirmed that a photocatalytic effect of the photoluminescent photocatalyst prepared according to the present invention is about twice as high as that of the general photocatalyst.

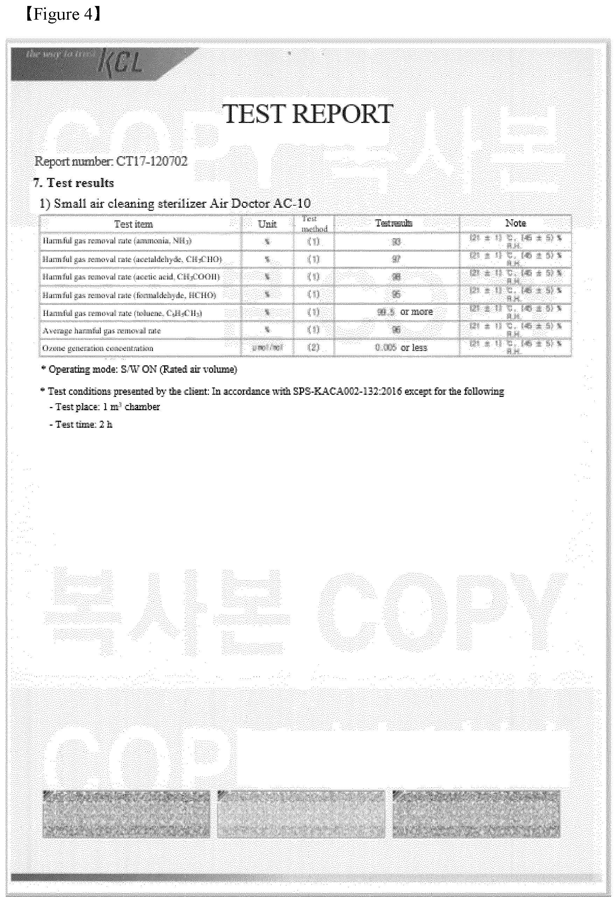

Furthermore, FIG. 4 shows the result of testing a harmful gas removal rate of an air purifier using the photoluminescent photocatalyst prepared according to the present invention, and it was confirmed that the average removal rates of ammonia gas, acetaldehyde, acetic acid, formaldehyde, toluene, and the like are 95% or more.

## Claims

1. A method of preparing photoluminescent photocatalyst beads for decomposition of harmful substances and removal of viruses, comprising:
ball-milling a mixture of a phosphor, zeolite, an inorganic binder, and titanium dioxide for 6 to 10 hours;
mixing 10 to 50 parts by weight of water with 100 parts by weight of the ball-milled mixture and kneading;
preparing the kneaded product in a form of beads through a bead filling machine;
drying the beads at 100 to 110 °C for 1 to 3 hours;
immersing the dried beads in a SiOz sol to coat the beads;
subjecting the coated beads to a first heat treatment at 400 to 500 °C for 1 to 2 hours;
primarily immersing the first heat-treated beads in a 10% sodium silicate solution;
subjecting the beads, which have been primarily immersed, to a second heat treatment at 100 to 150 °C for 1 hour to 2 hours;
secondarily immersing the second heat-treated beads in a 10% sodium silicate solution;
subjecting the beads, which have been secondarily immersed, to a third heat treatment at 100 to 150 °C for 1 hour to 2 hours;
immersing the third heat-treated beads in a TiOz sol to coat the beads with TiOz; and
subjecting the TiOz-coated beads to a fourth heat treatment at 400 to 500 °C for 1 to 2 hours.

2. The method of claim 1, wherein the phosphor is selected from the group consisting of CaAl₂O₄: Eu-based, SrAl₂O₄:Eu-based, BaAl₂O₄: Eu-based, ZnS: Cu-based, CaAl₂O₁₉: Eu-based, SrAl₂O₁₉: Eu-based, BaAl₂O₁₉: Eu-based, BaMgAl₁₀O₁₇: Eu-based, SrMgAAl₁₀O₁₇: Eu-based, and BaMg₂Al₁₀O₁₇: Eu-based phosphors.

3. The method of claim 1, wherein the mixture in the ball milling step is composed of 30 to 50% by weight of a phosphor, 30 to 50% by weight of zeolite, 10 to 20% by weight of an inorganic binder, and 1 to 5% by weight of TiOz.

4. The method of claim 1, wherein the SiOz sol coating solution is prepared by mixing tetraethyl orthosilicate(TEOS):ethanol:distilled water:nitric acid in a ratio of 2:13.5:11.5:0.3 parts by weight, mixing 0.8 parts by weight of polydimethylsiloxane (PDMS) based on 100 parts by weight of the mixture, stirring at 30 °C for 1 hour, and then aging for 24 hours.

5. The method of claim 1, wherein the TiOz coating includes coating with a TiO2 sol formed by mixing titanium isopropoxide (TTIP):ethanol:nitric acid:distilled water in a ratio of 2:13.5:0.3:11.5 parts by weight and stirring at 30 °C for 1 hour.

6. Photoluminescent photocatalyst beads for decomposition of harmful substances and removal of viruses, prepared according to the method of claim 1.
